(19) Europäisches Patentamt
European Patent Office
Office européen des brevets

(11) **EP 4 389 870 A1**

(12) **EUROPEAN PATENT APPLICATION**
published in accordance with Art. 153(4) EPC

(43) Date of publication:
**26.06.2024 Bulletin 2024/26**

(21) Application number: **23864155.9**

(22) Date of filing: **14.09.2023**

(51) International Patent Classification (IPC):
**C12M 1/12** *(2006.01)*     **C12N 5/00** *(2006.01)*
**B01J 2/06** *(2006.01)*

(52) Cooperative Patent Classification (CPC):
**B01J 2/06; C08J 3/12; C08J 5/00; C12M 1/12; C12N 5/00**

(86) International application number:
**PCT/KR2023/013771**

(87) International publication number:
**WO 2024/085449 (25.04.2024 Gazette 2024/17)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **17.10.2022 KR 20220133483**

(71) Applicant: **LG Chem, Ltd.**
**Seoul 07336 (KR)**

(72) Inventors:
• **OH, Seungjun**
  **Daejeon 34122 (KR)**
• **KIM, Minchae**
  **Daejeon 34122 (KR)**
• **KIM, Yeji**
  **Daejeon 34122 (KR)**
• **KIM, Myeongho**
  **Daejeon 34122 (KR)**
• **KIM, Jee Seon**
  **Daejeon 34122 (KR)**

(74) Representative: **Plasseraud IP**
**66, rue de la Chaussée d'Antin**
**75440 Paris Cedex 09 (FR)**

(54) **METHOD FOR PRODUCING MICROPARTICLES**

(57)   The present disclosure relates to a method for producing microparticles. The method for producing microparticles can produce microparticles of uniform size and narrow density range. Therefore, the method for producing microparticles can exhibit a higher production yield compared to a conventional method for producing microparticles. The microparticles have a density lower than that of water and their range is very narrow, and thus, when used as a microcarrier, they enable cell culture with high efficiency without problems floating up onto the surface of the culture medium during the cell culture process, and are easily separated and recovered after the culture process.

【FIG. 2】

EP 4 389 870 A1

## Description

### [TECHNICAL FIELD]

CROSS-REFERENCE TO RELATED APPLICATION(S)

This application claims priority benefit of Korean Patent Application No. 10-2022-0133483 filed on October 17, 2022 in the Korean Intellectual Property Office, the disclosure of which is incorporated herein by reference in its entirety.

The present disclosure relates to a method for producing microparticles.

### [BACKGROUND ART]

As the field of biopharmaceuticals and regenerative medicines expands, the demand for large-scale cell culture techniques that can efficiently produce cells, tissues, microorganisms, and the like is increasing. For example, the development of techniques for cultivating cells using a microcarrier has become active.

In microcarrier-related cell culture techniques, adherent cells are cultured using a microcarrier in a 3D bioreactor. Specifically, cells, a culture medium, and a microcarrier are placed in a bioreactor, the cells and the microcarrier are brought into contact with each other while stirring the culture medium, and the cells are allowed to adhere to the surface of the microcarrier and cultured. At this time, in order to be suitable for large-scale culture of cells, the microcarrier must have a high surface area/volume to which cells can adhere and proliferate.

On the other hand, currently commercially available microcarriers have a size of 100 to 300 $\mu$m and a density of about 1.1 to 1.3 g/cm$^3$. The density of cells cultured after adhering to the carrier is about 1.2 g/cm$^3$. Due to the density of the microcarrier and cells, it is advantageous to adhere the cells to the microcarrier at the initial stage of culturing the cells in the bioreactor, but it is difficult to apply centrifugation when separating and recovering cells after cultured. Therefore, in addition to centrifugation, a separate filtering method that can separate and recover cells based on the size of the microcarrier and cells must be used. However, filtering methods based on the size of the microcarrier and cells show problems that as the process is repeated, the filter becomes more clogged, the process takes longer, it causes physical damage and contamination of cells, and cells are lost during the filtering process. To solve such problems, it has been considered to produce a microcarrier using the properties of materials (polymers or polymeric components forming the microcarrier skeleton) with a density less than 1.0 g/cm$^3$ or greater than 1.3 g/cm$^3$. However, even in this case, the density range of the microcarrier that can be realized is limited, and thus, the adhesion between the microcarrier and the cells is not sufficient, and the culture efficiency is also not good. For example, when stirring a culture medium containing the microcarrier and cells, if the density of the microcarrier is too low, most of the microcarrier float on the surface of the culture medium despite stirring, and if the density of the microcarrier is too high, most of the microcarrier sink to the bottom of the culture medium despite stirring, which results in poor cell adhesion to the carrier and a decrease in culture efficiency.

Therefore, there is a need to develop a microcarrier-related technology that can be uniformly dispersed in the culture medium, which is advantageous for cell adhesion and culture, while also allowing easy separation and recovery of cells after cultured.

### [DETAILED DESCRIPTION OF THE INVENTION]

### [Technical Problem]

It is an object of the present disclosure to provide a method for producing microparticles.

### [Technical Solution]

A method for producing microparticles and microparticles produced therefrom according to specific embodiments of the present disclosure will be described below.

According to one embodiment of the present disclosure, there is provided a method for producing microparticles comprising the steps of: a) injecting a dispersed phase composition containing a polymerizable monomer into a continuous phase composition through a fine flow passage to generate a droplet comprised of the dispersed phase composition within the continuous phase composition; b) photopolymerizing the droplet; and c) thermally polymerizing the photopolymerized droplet.

As used herein, the term "microparticles" refers to particles having a size (diameter) of micro level, a particle group including such particles, or a particle group consisting of such particles. For example, a particle group including individual particles having a particle diameter in the range of 10 to 500 $\mu$m can be provided. The diameter can be measured in the same manner as described below.

As used herein, the term "dispersed phase composition" refers to a composition that can form a dispersed phase (or droplet) after being mixed with a continuous phase composition.

As used herein, the term "continuous phase composition" refers to a composition that can form a continuous phase after being mixed with a dispersed phase composition.

Unless otherwise specifically defined or explained herein, the temperature at which the production process is performed (or each production step) or the temperature at which the numerical characteristics of the produced particles are calculated or measured may be room temperature. Specifically, the term "room temperature" as used herein refers to a temperature under a condition which is not particularly increasing or decreasing, and may mean, for example, a temperature in the range of 15 to 30°C.

The present inventors have found through experiments that when the droplet produced with uniform size and shape through a specific method is continuously photopolymerized and thermally polymerized, it has high uniformity in shape and size, has a lower density characteristic than cells, has a very narrow density range, and can secure microparticles with excellent surface flatness at a high yield.

Now, the method for producing the microparticles of one embodiment will be described in detail.

The production method of the one embodiment comprises a) injecting a dispersed phase composition containing a polymerizable monomer into a continuous phase composition through a fine flow passage to generate a droplet comprised of the dispersed phase composition within the continuous phase composition.

In the step a) generating a droplet, the droplet of uniform size and shape can be produced by a specific method.

An oil in water (O/W) droplet, in which existing oil components are dispersed inside water, was formed by pouring a dispersed phase composition into a continuous phase composition and physically blending such as by stirring them. However, this method generates a droplet of non-uniform size, thereby providing microparticles that were not only non-uniform in size but also non-uniform in physical properties. On the other hand, in the step a) of generating a droplet, as the dispersed phase composition that forms the droplet is injected into the continuous phase composition through a fine flow passage to generate a droplet, it is possible to produce a droplet of uniform size.

Specifically, in the step a) of generating a droplet, a microfluidic device including a fine flow passage can be used.

As an example, the microfluidic device may comprise a first supply part through which the dispersed phase composition is supplied; a first flow passage through which the dispersed phase composition supplied from the first supply part can flow; a second supply part through which the continuous phase composition is supplied; a second flow passage through which the continuous phase composition supplied from the second supply part can flow; and a plurality of fine flow passages that connect side surfaces of the first and second flow passages to each other. A side surface of the flow passage refers to a direction that is not the flow direction of the fluid flowing within the flow passage.

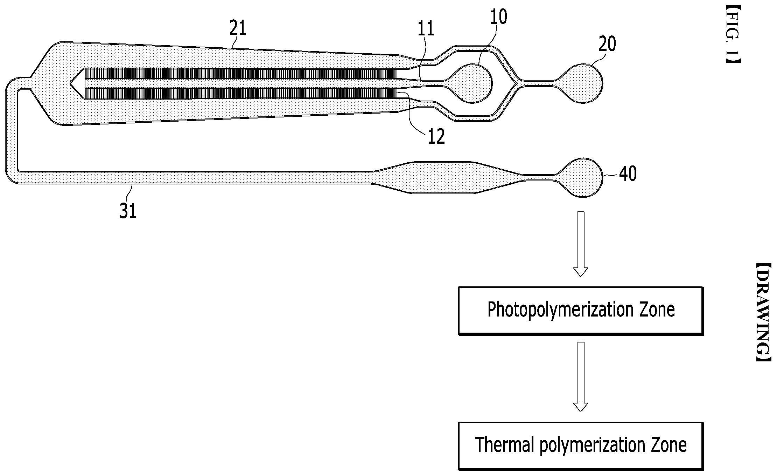

More specifically, referring to FIG. 1, the microfluidic device may include a first supply part 10 through which the dispersed phase composition is supplied; a first flow passage 11 through which the dispersed phase composition supplied from the first supply part can flow; a second supply part 20 through which the continuous phase composition is supplied; a second flow passage 21 through which the continuous phase composition supplied from the second supply part can flow; and a plurality of fine flow passages 12 that connect side surfaces of the first and second flow passages to each other. FIG. 1 is a diagram of a microfluidic device having a structure in which a first flow passage 11 through which the dispersed phase composition flows is arranged between two second flow passages 21 through which the continuous phase composition flows, and both side surfaces of the first flow passage 11 are connected to the side surfaces of the two second flow passages 21 through a plurality of fine flow passages 12, which is an example in which the plurality of fine flow passages 12 are arranged as densely as possible. The structure of the microfluidic device is not limited to that shown in FIG. 1, and can be freely modified with reference to FIG. 1 within a range that can achieve the purpose of the present disclosure.

The first flow passage and the second flow passage may be formed to be spaced apart from each other by the length of the fine flow passage having a desired length. The height and length of the fine flow passage are not particularly limited, and can be appropriately adjusted depending on the size of the desired droplet.

In the step a) of generating a droplet, the dispersed phase composition may be supplied to the first supply part of the microfluidic device, and the continuous phase composition may be supplied to the second supply part. The dispersed phase composition and the continuous phase composition may be injected into the first and second supply parts, respectively, through a pump, but are not limited thereto.

The dispersed phase composition supplied to the first supply part flows along the first flow passage, and the continuous phase composition supplied to the second supply part flows along the second flow passage. At this time, the speed of the dispersed phase composition may be adjusted from 1 $\mu\ell$/min to 10 m$\ell$/min. The speed of the continuous phase composition can be adjusted from 0.1 $\mu\ell$/min to 100 m$\ell$/min.

The dispersed phase composition flowing through the first flow passage flows to the second flow passage through a plurality of fine flow passages and meets the continuous phase composition flowing through the second flow passage.

The dispersed phase composition flowing from the first flow passage to the second flow passage through the plurality of fine flow passages generates a droplet at the boundary between the fine flow passage and the second flow passage, and the resulting droplet flows through the second flow passage together with the continuous phase composition.

The microfluidic device may further include a discharge part through which the droplet formed from the dispersed phase composition can be discharged. At this time, the microfluidic device may further include a third flow passage 31 that connects the second flow passage 21 and the discharge part 40 as shown in FIG. 1.

The dispersed phase composition is a precursor composition for forming low-density microparticles, and may be an oil phase that is insoluble in the continuous phase composition, which is a water phase.

Specifically, the dispersed phase composition may include a polymerizable monomer, a crosslinking agent, a low density oil, a photo-initiator, and a thermal initiator.

The polymerizable monomer may be a monomer having one or more unsaturated bonds. In one example, the polymerizable monomer may be a (meth)acrylate monomer or (meth)acrylamide monomer having a (meth)acryloyl group, or a vinyl-based monomer having a vinyl group, or a mixture thereof. In one example, the polymerizable monomer may include a styrene-based monomer as a vinyl-based monomer to form microparticles with a density lower than that of water.

The crosslinking agent can form a crosslinking structure with the polymerizable monomer or the prepolymer formed therefrom, thereby allowing the microparticles to maintain a spherical shape. When the microparticles have a spherical shape, it is possible to provide a microcarrier for cell culture that exhibits excellent cell culture efficiency due to a large specific surface area. As an example, an ethylenically unsaturated crosslinking agent having two or more unsaturated bonds may be used as the crosslinking agent. More specifically, considering the density of microparticles, the cell culture process, and the like, it may be preferable to use, for example, divinylbenzene as the crosslinking agent.

The dispersed phase composition may contain the crosslinking agent in an amount of 10 parts by weight or more, 50 parts by weight or more, 100 parts by weight or more, 200 parts by weight or more, 250 parts by weight or more, or 300 parts by weight or more, and 1000 parts by weight or less, 800 parts by weight or less, 700 parts by weight or less, 600 parts by weight or less, 500 parts by weight or less, or 450 parts by weight or less based on 100 parts by weight of the polymerizable monomer.

Within this range, the microparticles can have a particle density suitable for cell culture and centrifugation while stably maintaining a spherical shape.

The low density oil is included in the dispersed phase composition such that the microparticles have a density lower than that of water. As used herein, the term "low density oil" refers to a hydrocarbon oil that has a lower density than that of water at room temperature. For example, the low density oil refers to a hydrocarbon oil having a density of 0.700 to 0.997 $g/cm^3$ at 21°C.

In the prior art, expanded styrene particles were produced using a blowing agent to lower the density of microcarrier particles, but when using a blowing agent, the distribution range of particle diameter and density becomes excessively wide, and thus, it was not easy to obtain a microcarrier having a diameter and density suitable for cell culture purposes in sufficient yield. However, in the production method of the one embodiment, low density microparticles can be provided without a blowing process by using a dispersed phase composition containing low density oil. That is, the microparticles produced by the above production method are non-expanded particles.

The low density oil may escape from the droplet during the polymerization process, and a part thereof is trapped within the polymer, which contributes to lowering the density of microparticles. Furthermore, the microparticles obtained using a low density oil do not sink to the bottom of the culture medium or float on the surface of the culture medium, and can maintain a state of being uniformly dispersed in the culture medium. As a result, when the microparticles are used as the microcarrier for cell culture, the degree of suspension of the microcarrier in the culture medium can be improved, the adhesion between the microcarrier and cells can be increased, and the culture efficiency can also be increased.

The type of the low density oil that can be included in the dispersed phase composition is not particularly limited, but can be selected in consideration of ease of implementation of the production method or ensuring the characteristics of microparticles described below. For example, a low density hydrocarbon oil in which the upper limit of the density is 0.800 $g/cm^3$ or less or 0.790 $g/cm^3$ or less can be used. At this time, the lower limit of density of the low density hydrocarbon oil is not particularly limited, but may be, for example, 0.750 $g/cm^3$ or more or 0.760 $g/cm^3$ or more.

In one example, the low density oil may include one or more linear or branched saturated hydrocarbon compounds having 8 to 50 carbon atoms. Specifically, the low density oil may include, for example, a normal alkane having 8 to 16 carbon atoms, an isoalkane having 8 to 16 carbon atoms, or a mixture thereof. As a specific example, dodecane having 12 carbon atoms, hexadecane having 16 carbon atoms, or Isopar M (a mixture of an isoalkane having 12 to 14 carbon atoms and an isoalkane having 13 to 16 carbon atoms) can be used as the low density oil.

The dispersed phase composition may contain the low density oil in an amount of 5 parts by weight or more, 10 parts by weight or more, 20 parts by weight or more, 30 parts by weight or more, 40 parts by weight or more, or 50 parts by weight or more, and 100 parts by weight or less, 97 parts by weight or less, 95 parts by weight or less, 94 parts by weight or less, 93 parts by weight or less, or 92 parts by weight or less based on 100 parts by weight of the polymerizable monomer.

When used as a microcarrier for cell culture within this range, it can maintain a state of being uniformly dispersed in the culture medium without sinking to the bottom of the culture medium or floating on the surface of the culture medium, thereby providing microparticles with appropriate density that exhibit high cell adhesion rate and culture efficiency, and can be easily separated and recovered by centrifugation after culturing the cells.

The production method of the one embodiment produces microparticles through a continuous photopolymerization process and a thermal polymerization process. Thereby, the dispersed phase composition includes both a photo-initiator and a thermal initiator.

The types of the photo-initiator and the thermal initiator are not particularly limited, and various initiators known in the art can be used as long as they do not impede securing the particle characteristics of the microparticles described above.

As the photo-initiator, for example, one or more selected from the group consisting of benzoin ether, dialkyl acetophenone, hydroxyl alkylketone, phenyl glyoxylate, benzyl dimethyl ketal, acyl phosphine and α-aminoketone can be used. On the other hand, as a specific example of acylphosphine, commercially available IRGACURE 819, i.e., bis(2,4,6-trimethylbenzoyl)-phenylphosphineoxide, can be used.

The photo-initiator may be contained in an amount of 0.1 parts by weight or more, 1 part by weight or more, 3 parts by weight or more, 5 parts by weight or more, 10 parts by weight or more, or 15 parts by weight or more, and 30 parts by weight or less, 27 parts by weight or less, 25 parts by weight or less, 23 parts by weight or less, 20 parts by weight or less, or 18 parts by weight or less based on 100 parts by weight of the polymerizable monomer.

Within this range, microparticles can be polymerized at an appropriate rate and may exhibit desired properties.

Further, as the thermal initiator, for example, one or more initiators selected from the group consisting of a persulfate-based initiator, an azo-based initiator, hydrogen peroxide, and ascorbic acid can be used.

Specifically, examples of the persulfate-based initiator include sodium persulfate (NazSzOs), potassium persulfate ($K_2S_2O_8$), ammonium persulfate (($NH_4$)$_2S_2O_8$), and the like. Examples of the azo-based initiator include 2,2-azobis-(2-amidinopropane) dihydrochlorid, 2,2-azobis-(N,N-dimethylene)isobutyramidine dihydrochloride, 2-(carbamoylazo)isobutyronitrile, 2,2-azobis[2-(2-imidazolin-2-yl)propane]dihydrochloride, 4,4-azobis-(4-cyanovaleric acid), 2,2-azobis(2,4-dimethylvaleronitrile) (brand name: V-65), and the like.

The thermal initiator may be contained in an amount of 0.1 part by weight or more, 0.5 part by weight or more, 1 part by weight or more, 1.5 parts by weight or more, 2 parts by weight or more, 2.5 parts by weight or more, or 3 parts by weight or more, and 30 parts by weight or less, 25 parts by weight or less, 20 parts by weight or less, 15 parts by weight or less, 10 parts by weight or less, 8 parts by weight or less, or 6 parts by weight or less, based on 100 parts by weight of the polymerizable monomer.

Within this range, microparticles can be polymerized at an appropriate rate and may exhibit desired properties.

In addition to the components described above, the dispersed phase composition may include various additives known in the art within the scope of not impairing the purpose of the present disclosure.

On the other hand, the continuous phase composition may be a water phase. Specifically, the continuous phase composition may be an aqueous solution containing a surfactant and water.

The water is not particularly limited, but may be distilled water or deionized water.

The surfactant may be an ionic surfactant or a non-ionic surfactant. For example, the ionic surfactant may be sodium dodecyl sulfate (SDS), and the nonionic surfactants may be Tween 20, Tween 40, Tween 60, Tween 80, Triton X-100, polyvinyl alcohol (PVA), polyethylene glycol (PEG), and the like.

The continuous phase composition may contain the surfactant in an amount of 0.01 wt.% or more, 0.05 wt.% or more, 0.1 wt.% or more, 0.2 wt.% or more, or 0.3 wt.% or more, and 5 wt.% or less, 4 wt.% or less, 3 wt.% or less, 2 wt.% or less, or 1 wt.% or less, based on the overall continuous phase composition. It is advantageous to produce microparticles with desired properties within this range.

On the other hand, the droplet produced in the step a) of generating a droplet may be introduced into the step b) of photopolymerizing the droplet. At this time, the droplet may be mixed with further continuous phase composition before being introduced into the photopolymerization process.

In one example, a suspension containing droplets discharged at the discharge port of a microfluidic device can be mixed with further continuous phase composition and then introduced into a photopolymerization process. Through this process, the shape of microparticles can be uniformly controlled.

The further continuous phase composition may be of the same composition as the continuous phase composition used in the step a) of generating the droplets, or may be of a different composition. As an example, in the step a) of generating a droplet, an aqueous sodium dodecyl sulfate solution is used as the continuous phase composition, and the suspension containing the droplet may be mixed with an aqueous polyvinyl alcohol solution. However, without being limited thereto, the droplet generated in the step a) of generating a droplet can be immediately transferred to the photopolymerization process.

In the photopolymerization step b), the droplets produced in the step a) are photopolymerized to produce pre-microparticles. In the photopolymerization step b), the droplets can be irradiated with UV using an ultraviolet lamp to induce photopolymerization of the droplet. Referring to Comparative Example 3 described below, even if sufficient UV is irradiated in the photopolymerizing step b), complete polymerization and crosslinking of the droplet are not achieved, so that solidified particles cannot be obtained. It can be confirmed that complete polymerization and crosslinking are achieved through the subsequent thermal polymerization step c).

The photopolymerized droplet that has undergone the photopolymerization step b) can be introduced into the thermal polymerization step c), whereby complete polymerization and crosslinking can be achieved. As an example, the thermal polymerization may be performed at 40°C or more, 45°C or more, 50°C or more, 55°C or more, or 60°C or more, and 100°C or less, 90°C or less, 80°C or less, 75°C or less, or 70°C or less. Further, the thermal polymerization time can be controlled to 1 hour or more, 2 hours or more, 2.5 hours or more, 3 hours or more, 3.5 hours or more, or 4 hours or more, and 48 hours or less, 36 hours or less, 24 hours or less, 12 hours or less, 10 hours or less, or 8 hours or less. When the thermal polymerization temperature and time are controlled within the above-mentioned range, microparticles having uniform shape and size can be provided without particle agglomeration.

In the method for producing microparticles according to the one embodiment, the droplet production step, the photopolymerization step and the thermal polymerization step are performed in a continuous process, thereby facilitating the production method and exhibiting excellent productivity.

When the above microparticles are used as a microcarrier, the production method of one embodiment may further comprise forming a primer layer and/or a cell adhesion inducing layer on the surface of the microparticles.

The primer layer makes it possible to introduce a functional polymer onto the surface of microparticles that do not have functional groups, and functions as a so-called adhesive layer. For example, the cell adhesion inducing layer or cells can be stably maintained on the particle via a primer layer.

Although not particularly limited, a catechol derivative that can induce aqueous phase adhesion can be used as a compound that can form a primer layer. For example, one or more selected from the group consisting of L-dihydroxyphenylalanine (L-DOPA), dopamine, norepinephrine, epinephrine, epigallocatechin, and derivatives thereof can be used in the formation of a primer layer. The primer layer formed including the above compound imparts hydrophilicity to the surface of the particles, thereby further enhancing the dispersibility of the particles in an aqueous dispersion that is a continuous phase composition.

In one illustrative example, the ratio between the radius of the microparticles and the thickness of the primer layers may be 1:0.00001 to 1:0.01, or 1:0.0001 to 1:0.001. If the ratio between the radius of the microparticles and the thickness of the primer layers is too low, the primer layer is too thin compared to the microparticles, and thus, the effect of modifying the surface of the microparticles to be hydrophilic is minimal. If the ratio is too high, the primer layers become thicker relative to the microparticles, so that the adhesion efficiency of cells and microparticles may decrease during the cell culture.

The cell adhesion inducing layer is composed of cell adhesion materials, which serve to provide a site where cell transmembrane proteins can bind. Thereby, adherent cells can stably adhere, spread, and culture. Although not particularly limited, one or more selected from the group consisting of gelatin, collagen, fibronectin, chitosan, polydopamine, tannic acid, polyphenol, poly L-lysine, vitronectin, peptides including RGD, lignin, cationic dextran and their derivatives can be used as a compound for forming a cell adhesion inducing layer. In addition, the cell adhesion inducing layer formed including the above compound can modify the surface of the microparticles to be hydrophilic and improve the water dispersibility of the microparticles.

The method for producing microparticles according to the one embodiment may further include a cleaning step after the thermal polymerization step c). Impurities unrelated to microparticles can be removed by cleaning. The cleaning method is not particularly limited, and any known cleaning method can be used. For example, the cleaning may be performed by adding microparticles to alcohol such as ethanol and stirring the mixture. Although not particularly limited, this cleaning may be repeated, for example, three or more times. In one example, the cleaning may be performed after forming a primer layer and/or a cell adhesion inducing layer onto the surface of the microparticles.

The method for producing microparticles according to the one embodiment may further include a drying step after the cleaning. Residual solvents, and the like can be removed through drying. The drying method is not particularly limited, and any known drying method can be used. For example, the drying can be performed using an oven or at room temperature. Further, although not particularly limited, the drying may be performed under vacuum.

The production method of one embodiment can produce microparticles with uniform size and shape and a narrow density range. In the case of a conventional method for producing microparticles, the production yield was low because after particle formation, particles with desired shapes and physical properties were classified from particles with undesirable shapes and physical properties, but the method for producing microparticles can obtain microparticles having a desired shape and physical properties without going through such classification work, and can exhibit a high production yield.

The microparticles produced by the production method of the one embodiment have a size at the micrometer level. In one example, the microparticles may have a diameter of 10 $\mu$m or more, 15 $\mu$m or more, 20 $\mu$m or more, 22 $\mu$m or more, 25 $\mu$m or more, 27 $\mu$m or more, 30 $\mu$m or more, or 35 $\mu$m or more, and 500 $\mu$m or less, 400 $\mu$m or less, 300 $\mu$m or less, 200 $\mu$m or less, 100 $\mu$m or less, 80 $\mu$m or less, 60 $\mu$m or less, or 50 $\mu$m or less. The diameter can be calculated by determining the two-dimensional (2D) plane area of the particle through an optical microscope, and then back calculating the equation ($S=\pi r^2$) relating to the plane area. At this time, the diameter may be the arithmetic average of at least 100 particle diameter values. As the microparticles have a diameter within the above range, they have a surface area suitable for cell culture and can exhibit good cell culture efficiency.

As the microparticles have a very uniform size and shape, the coefficient of variation in the diameter may be of very small values. As an example, the coefficient of variation in diameter of the microparticles may be 10% or less, 8% or less, 6% or less, or 5% or less. At this time, the coefficient of variation in the diameter may be a value calculated for at least 100 or more particles. Uniform particle size provides an optimal surface for cell growth and may contribute to improved culture efficiency. The lower limit of the coefficient of variation in diameter of the microparticles is not particularly limited, but may be, for example, 0% or more.

The density of conventional commercially available microcarriers used for cell culture having a density of about 1.2 g/cm$^3$ is at the level of about 1.1 to 1.3 g/cm$^3$. Although the microcarrier with such a density is advantageous for initial cell adhesion, there is a problem that cell separation and recovery through centrifugation after cultured is difficult.

Microparticles produced by the production method of the one embodiment have a density lower than that of water. Therefore, when separating and recovering cultured cells from a microparticle used as a microcarrier, the difference in sedimentation rate due to gravity may be greater than when using a conventional microcarrier, and therefore, it becomes possible to separate microparticles and cells more easily.

On the other hand, if the density of the microcarrier is too low, it is difficult for cells to adhere to the microcarrier at the initial stage of cell culture, and during the cell culture process, the microcarrier floats in the culture medium, which reduces culture efficiency.

The microparticles produced by the production method of the one embodiment may have a density of 0.985 g/cm$^3$ or more and less than 0.998 g/cm$^3$ at 15 to 25°C. The density of the microparticles can be confirmed by preparing a solution having a predetermined density (e.g., deionized water or ethanol aqueous solution), mixing the particles to be confirmed, and determining whether or not they float. Specifically, the density being 0.985 g/cm$^3$ or more and less than 0.998 g/cm$^3$ at 15 to 25 °C means that there are no floating particles in an ethanol aqueous solution with a density of 0.985 g/cm$^3$ at 15 to 25°C, and that there are no particles sedimented in deionized water with a density of 0.998 g/cm$^3$ at 15 to 25°C.

The microparticles may have a narrower range of density distribution. As an example, the microparticles may have a density of 0.985 g/cm$^3$ or more or 0.990 g/cm$^3$ or more, and less than 0.998 g/cm$^3$, 0.995 g/cm$^3$ or less, or 0.990 g/cm$^3$ or less at 15 to 25°C. As a more specific example, the microparticles may have a density of 0.985 g/cm$^3$ or more and 0.990 g/cm$^3$ or less, or 0.990 g/cm$^3$ or more and 0.995 g/cm$^3$ or less at 15 to 25°C, which may have a narrow range of density distribution.

The microparticles produced by the production method of the one embodiment may have a spherical shape. The spherical shape generally means having a shape close to a sphere, which can be confirmed with the naked eye, but for example, it may mean that the sphericity value calculated by Equation 1 below is about 0.80 or more. If the particles have a spherical shape, a large surface area can be secured, and cell adhesion performance when used as a microcarrier can be improved.

[Equation 1]

$$\text{Sphericity} = \frac{\pi^{\frac{1}{3}}(6V_p)^{\frac{2}{3}}}{A_p}$$

(wherein, in Equation 1, $V_p$ is the volume of the particles, and $A_p$ is the surface area of the particles.)

The microparticles produced by the production method of the one embodiment can be used as a microcarrier for cell culture. At this time, the type of the cells is not particularly limited. For example, the cells can be adherent animal cells, specifically a cell such as a fibroblast, chondrocyte, mesenchymal stem cell, CHO, HEK 293, Vero cell, BHK21 or MDCK.

The cells can be cultured by adhering to microparticles in the culture medium. The culture medium may contain nutrients close to the conditions of living organisms based on body fluids such as plasma or lymph, and various additives to sufficiently satisfy environmental conditions such as pH, temperature, and osmotic pressure. As such additives, various materials widely known in the field of cell culture-related techniques can be used without limitation.

And, microparticles and cells may have a density lower than that of the culture medium. Specifically, the microparticles may have a density of 0.985 g/cm$^3$ or more and less than 0.998 g/cm$^3$ at 15 to 25°C, and the cells may have a density in the range of 1.10 to 1.25 g/cm$^3$ (about 1.2 g/cm$^3$). Therefore, the microparticles injected into the culture medium may float in the culture medium, and as the number of cells adhered to the surface of the floating microparticles gradually increases, the density of the microparticles to which the cells are adhered also gradually increases and may sink to the bottom of the container containing the culture medium. And, after culturing cells, the cultured cells can be secured by separating the cells from the microparticle-cell complex through centrifugation.

**[Advantageous Effects]**

A method for producing microparticles according to one embodiment of the disclosure can produce microparticles having uniform size and narrow density range. Therefore, the method for producing microparticles can exhibit a higher production yield compared to existing methods for producing microparticles. The microparticles have a density lower than that of water and their range is very narrow, and thus, when used as a microcarrier, they enable cell culture with high efficiency without problems floating up onto the surface of the culture medium during the cell culture process, and are easily separated and recovered after the culture process.

[BRIEF DESCRIPTION OF THE DRAWINGS]

FIG. 1 is a schematic diagram of a device capable of realizing a method for producing microparticles according to an embodiment.
FIG. 2 is an SEM image of microparticles produced in Example 1.
FIG. 3 is an SEM image of microparticles produced in Example 2.
FIG. 4 is an SEM image of microparticles produced in Comparative Example 1.
FIG. 5 is an SEM image of microparticles produced in Comparative Example 3.
FIGS. 6 and 7 are photographs taken to observe the degree of suspension of the particles after mixing the microparticles produced in Examples 1 and 2 with deionized water having a density of 0.998 g/cm$^3$ and aqueous ethanol solutions having densities of 0.995 g/cm$^3$, 0.990 g/cm$^3$ and 0.985 g/cm$^3$, respectively. The numbers above the photos in FIGS. 6 and 7 indicate density, and the unit is g/cm$^3$.

<Description of Reference Numerals>

10: first supply part
11: first flow passage
12: fine flow passage
20: second supply part
21: second flow passage
31: third flow passage
40: discharge part

[DETAILED DESCRIPTION OF THE EMBODIMENTS]

Hereinafter, actions and effects of the disclosure will be described in more detail with reference to specific examples of the disclosure. However, these examples are for illustrative purposes only, and the scope of the disclosure is not limited thereby in any way.

Example 1: Production of microparticles

400 parts by weight of divinylbenzene and 68 parts by weight of Isopar M (low density oil) based on 100 parts by weight of styrene were mixed to prepare a mixture. 17 parts by weight of IRGACURE 819 (photo-initiator) and 5.7 parts by weight of V-65 (thermal initiator) based on 100 parts by weight of styrene were added to the resulting mixture to prepare a dispersed phase composition.

SDS (sodium dodecyl sulfate) was dissolved in deionized water at a concentration of 0.5 wt.% to prepare a continuous phase composition.

The dispersed phase composition and the continuous phase composition prepared previously were injected into a first supply part 10 and a second supply part 20 of the microfluidic device respectively, through a pump (not shown) as shown in FIG. 1. The dispersed phase composition injected into the first supply part 10 flows along the first flow passage 11, and is supplied to the second flow passage 21 via a fine flow passage 12 to form a droplet within the continuous phase composition. The suspension containing the droplet was discharged through the discharge part 40.

The suspension containing the droplets was then supplied to a mixing zone and mixed with a 4 wt.% polyvinyl alcohol (PVA) aqueous solution supplied by a separate pump via a T-connector. A static mixer was used in the mixing zone.

The mixture mixed in the mixing zone was transferred to the photopolymerization zone. In the photopolymerization zone, the droplet was irradiated with UV light using a UV lamp to induce a photopolymerization reaction, and the droplet was photopolymerized for about 10 minutes.

Then, the photopolymerized droplet transferred to the thermal polymerization zone. In the thermal polymerization zone, the photopolymerized droplet was thermally polymerized at 65°C for 4 hours.

After the thermal polymerization, the produced particles were washed three times with deionized water and again three times with ethanol to obtain microparticles.

Example 2: Production of microparticles

Microparticles were produced in the same manner as in Example 1, except that 400 parts by weight of divinylbenzene, 89 parts by weight of Isopar M, 17.7 parts by weight of IRGACURE 819 (photo-initiator), and 5.9 parts by weight of V-65 (thermal initiator) based on 100 parts by weight of styrene were used.

Comparative Example 1: Production of microparticles

33 parts by weight of divinylbenzene and 34 parts by weight of Isopar M based on 100 parts by weight of styrene were mixed to prepare a mixture.

3.5 parts by weight of benzoyl peroxide and 0.5 parts by weight of tert-butyl peroxy benzoate based on 100 parts by weight of styrene were added to the resulting mixture to prepare a dispersed phase composition.

2.5 g of polyvinyl alcohol (PVA) having a weight average molecular weight in the range of 85,000 to 125,000 and a hydrolysis rate of 87 to 89% was dissolved in 250 g of distilled water to prepare a continuous phase composition.

50 g of a 1 wt.% polyvinyl alcohol (PVA) aqueous solution was mixed with the dispersed phase composition, and the mixture was stirred in an oil bath until a uniform dispersion was obtained. Specifically, the oil bath was gradually heated at room temperature while stirring at 800 rpm, and suspension polymerization was carried out at a temperature of 85 to 88°C and a speed of 600 to 800 rpm. The polymerization was carried out under nitrogen purge.

After 6 hours of reaction, the produced particles were recovered through a 100 $\mu$m sieve, washed 5 times with ethanol, and dried at room temperature.

Comparative Example 2: Production of microparticles

Microparticles were produced in the same manner as in Example 1, except that it did not go through a photopolymerization process in Example 1.

Comparative Example 3: Production of microparticles

Microparticles were produced in the same manner as in Example 1, except that it did not go through a thermal polymerization process in Example 1.

Test Example: Evaluation of the shape and physical properties of microparticles

(1) Evaluation of particle shape

The shape of the microparticles was observed through a scanning electron microscope (SEM).

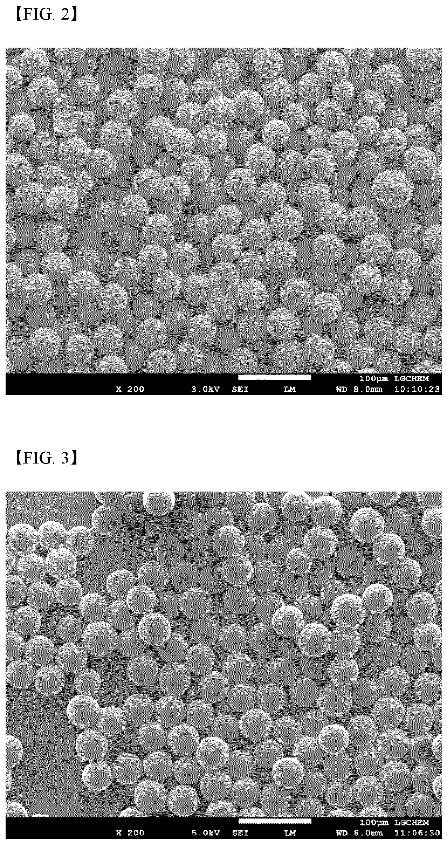

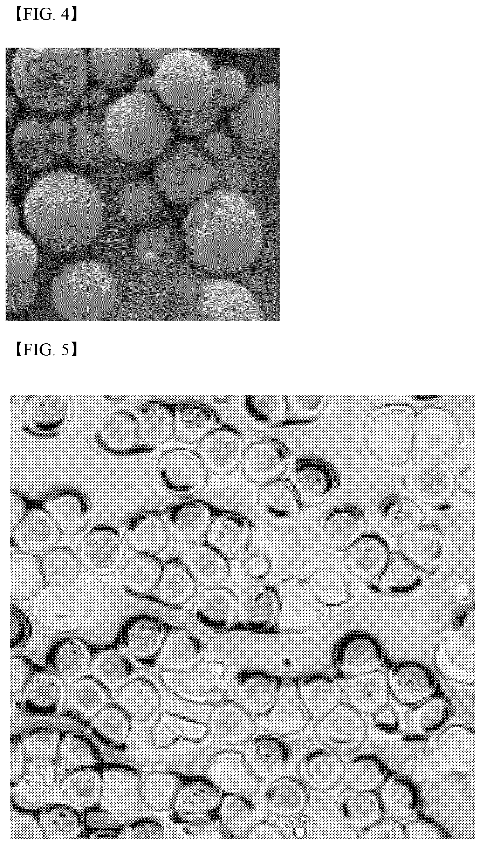

As confirmed through SEM images, Examples 1 and 2 provided spherical particles whose surface was uniform with very uniform size (see FIGS. 2 and 3, respectively), but Comparative Example 1 provided particles in which particle size was non-uniform and particle surface was non-uniform (see FIG. 4). In Comparative Example 2, a phenomenon in which droplets gather together (coalescence) occurred due to a sudden increase in the polymerization temperature, and in Comparative Example 3, solidified particles were not formed (see FIG. 5).

(2) Evaluation of diameter and its coefficient of variation

The diameters of 100 particles were measured through an optical microscope, and the arithmetic mean value for the measured diameter was determined. Specifically, the diameter of each individual particle was calculated by determining the two-dimensional (2D) plane area of the particles through an optical microscope and then back calculating the equation ($S=\pi r^2$) relating to the plane area. Further, the coefficient of variation was determined from the diameters of 100 particles.

In the case of Example 1, the particle diameter was 42.3 $\pm$ 1.9 $\mu$m, and the coefficient of variation in diameter was about 4.4%, but in the case of Comparative Example 1, the particle diameter was 130 $\pm$ 33 $\mu$m, and the coefficient of variation in diameter was about 25.4%.

(3) Evaluation of density

The microparticles produced in Examples 1 and 2 were respectively added to deionized water having a density of 0.998 g/cm$^3$ and ethanol aqueous solutions having densities of 0.995 g/cm$^3$, 0.990 g/cm$^3$ and 0.985 g/cm$^3$ under the conditions of room temperature (about 21°C) and ambient pressure (1 atm). After the addition, the vial containing each solution was capped, shaken vigorously, and after about 5 minutes, it was confirmed whether the particles were floating.

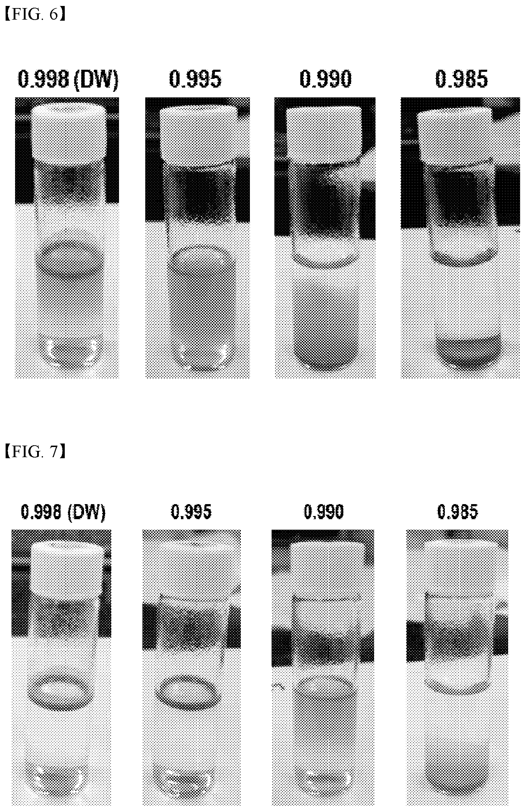

In the case of Example 1, as shown in FIG. 6, in deionized water with a density of 0.998 g/cm$^3$, all particles floated without sedimented particles; in a solution with a density of 0.995 g/cm$^3$, a significant number of particles floated and some particles were dispersed; in a solution with a density of 0.990 g/cm$^3$, most particles were sedimented and some particles were dispersed; and in a solution with a density of 0.985 g/cm$^3$, all particles were sedimented. As a result, it was confirmed that the microparticles of Example 1 had a very narrow density distribution of 0.990 to 0.995 g/cm$^3$.

In the case of Example 2, as shown in FIG. 7, in deionized water with a density of 0.998 g/cm$^3$ and a solution with a density of 0.995 g/cm$^3$, all particles were floated without sedimented particles; in a solution with a density of 0.990 g/cm$^3$, most particles were suspended and some particles were dispersed; and in a solution with a density of 0.985 g/cm$^3$, all particles were sedimented. As a result, it was confirmed that the microparticles of Example 2 had a very narrow density distribution of 0.985 to 0.990 g/cm$^3$.

On the other hand, in the case of the microparticles of Comparative Example 1, it was confirmed that they were floated in a solution with a density of 0.990 g/cm$^3$ and sedimented in a solution with a density of 0.950 g/cm$^3$, which resulted in a very wide density distribution of 0.950 to 0.990 g/cm$^3$.

Through the above test examples, it was confirmed that the microparticles produced according to the production method of microparticles of one embodiment of the disclosure were uniform in the surface and very uniform in the size and shape, and had a very narrow density range. In particular, the desired low density particles can be obtained in high yield due to high uniformity in size and shape and very narrow density range.

In the case of Comparative Example 1 produced through a suspension polymerization process, there is a problem that particles having a very low density float up onto the surface of the culture medium during the cell culture process. On the other hand, it is confirmed that the particles produced in Example 1 had a density of 0.990 to 0.995 g/cm$^3$, the particles produced in Example 2 had a density of 0.985 to 0.990 g/cm$^3$, which enabled high efficiency cell culture without problems floating up onto the surface of the culture medium during the cell culture process, and microcarriers that can be easily separated and recovered after the culture process can be provided.

**Claims**

1. A method for producing microparticles comprising the steps of:

   a) injecting a dispersed phase composition containing a polymerizable monomer into a continuous phase composition through a fine flow passage to generate a droplet comprised of the dispersed phase composition within the continuous phase composition;
   b) photopolymerizing the droplet; and
   c) thermally polymerizing the photopolymerized droplet.

2. The method as claimed in claim 1, wherein the a) injecting the dispersed phase composition to generate a droplet utilizes a microfluidic device which comprises a first supply part through which the dispersed phase composition is supplied; a first flow passage through which the dispersed phase composition supplied from the first supply part can flow; a second supply part through which the continuous phase composition is supplied; a second flow passage through which the continuous phase composition supplied from the second supply part can flow; and a plurality of the fine flow passages that connect side surfaces of the first flow passage and the second flow passage to each other.

3. The method as claimed in claim 1, wherein the dispersed phase composition comprises the polymerizable monomer, a crosslinking agent, a low density oil, a photo-initiator, and a thermal initiator.

4. The method as claimed in claim 3, wherein the crosslinking agent is contained in an amount of 10 to 1000 parts by weight based on 100 parts by weight of the polymerizable monomer.

5. The method as claimed in claim 3, wherein the low density oil is contained in an amount of 5 to 100 parts by weight based on 100 parts by weight of the polymerizable monomer.

6. The method as claimed in claim 3, wherein the photo-initiator is contained in an amount of 0.1 to 30 parts by weight based on 100 parts by weight of the polymerizable monomer.

7. The method as claimed in claim 3, wherein the thermal initiator is contained in an amount of 0.1 to 30 parts by weight based on 100 parts by weight of the polymerizable monomer.

8. The method as claimed in claim 1, wherein the continuous phase composition comprises a surfactant and water.

9. The method as claimed in claim 1, further comprising mixing the droplet with a further continuous phase composition after the a) generating the droplets.

10. The method as claimed in claim 1, wherein the microparticles are used as a microcarrier for cell culture.

11. The method as claimed in claim 1, which produces microparticles with a diameter of 10 to 500 $\mu$m.

12. The method as claimed in claim 1, which produces microparticles with a coefficient of variation in diameter is 10% or less.

13. The method as claimed in claim 1, which produces microparticles with a density of 0.985 g/cm$^3$ or more and less than 0.998 g/cm$^3$ at 15 to 25°C.

【DRAWING】

【FIG. 1】

【FIG. 2】

【FIG. 3】

【FIG. 4】

【FIG. 5】

【FIG. 6】

| 0.998 (DW) | 0.995 | 0.990 | 0.985 |

【FIG. 7】

| 0.998 (DW) | 0.995 | 0.990 | 0.985 |

## INTERNATIONAL SEARCH REPORT

| International application No. |
| --- |
| **PCT/KR2023/013771** |

| A. | CLASSIFICATION OF SUBJECT MATTER |
| --- | --- |

**C12M 1/12**(2006.01)i; **C12N 5/00**(2006.01)i; **B01J 2/06**(2006.01)i

According to International Patent Classification (IPC) or to both national classification and IPC

| B. | FIELDS SEARCHED |
| --- | --- |

Minimum documentation searched (classification system followed by classification symbols)

C12M 1/12(2006.01); A61K 35/44(2006.01); A61K 9/50(2006.01); B01J 13/00(2006.01); B01J 2/02(2006.01);
C08F 2/38(2006.01); C08F 220/06(2006.01); C08F 220/26(2006.01); C08J 3/00(2006.01); C12M 3/00(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 미세유체(microfluidic), 광중합(photo polymerization), 열중합(thermal polymeriz ation), 마이크로 입자(micro particle), 세포 배양(cell culture)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| A | KR 10-2020-0127867 A (SOOKMYUNG WOMEN'S UNIVERSITY INDUSTRY-ACADEMIC COOPERATION FOUNDATION) 11 November 2020 (2020-11-11)<br>See claims 1 and 8. | 1-13 |
| A | WO 2018-165584 A1 (UNIVERSITY OF FLORIDA RESEARCH FOUNDATION) 13 September 2018 (2018-09-13)<br>See claims 12-25. | 1-13 |
| A | JP 2021-151210 A (TOYO INK SC HOLDINGS CO., LTD.) 30 September 2021 (2021-09-30)<br>See entire document. | 1-13 |
| A | KR 10-2014-0069499 A (POSTECH RESEARCH AND BUSINESS DEVELOPMENT FOUNDATION) 10 June 2014 (2014-06-10)<br>See entire document. | 1-13 |
| A | US 2019-0105279 A1 (AUBURN UNIVERSITY) 11 April 2019 (2019-04-11)<br>See entire document. | 1-13 |

☐ Further documents are listed in the continuation of Box C.    ☑ See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **18 December 2023** | **18 December 2023** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**
Information on patent family members

International application No.

**PCT/KR2023/013771**

| Patent document cited in search report | | | Publication date (day/month/year) | Patent family member(s) | | | Publication date (day/month/year) |
|---|---|---|---|---|---|---|---|
| KR | 10-2020-0127867 | A | 11 November 2020 | KR | 10-2425901 | B1 | 28 July 2022 |
| WO | 2018-165584 | A1 | 13 September 2018 | EP | 3592787 | A1 | 15 January 2020 |
| | | | | EP | 3592787 | A4 | 20 January 2021 |
| | | | | JP | 2020-509746 | A | 02 April 2020 |
| | | | | US | 2020-0010798 | A1 | 09 January 2020 |
| JP | 2021-151210 | A | 30 September 2021 | None | | | |
| KR | 10-2014-0069499 | A | 10 June 2014 | KR | 10-1443981 | B1 | 14 October 2014 |
| US | 2019-0105279 | A1 | 11 April 2019 | US | 11166920 | B2 | 09 November 2021 |
| | | | | US | 2022-0125735 | A1 | 28 April 2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020220133483